# EUROPEAN PATENT APPLICATION

(11) **EP 4 316 839 A1**
(43) Date of publication of application: **07.02.2024**
(21) Application number: 22306177.1
(22) Date of filing: 03.08.2022
(51) Int. Cl.: B33Y 70/00, B29C 64/106, B29C 64/209, C12M 1/00, C12M 3/00, C12M 1/26

(54) **PRINTING NEEDLE HOLDER DEVICE, STERILE ENCLOSURE AND METHOD OF PRINTING AND CULTURING BIOLOGICAL CELLS**

(71) Applicant: SARTORIUS STEDIM FMT SAS, 13400 Aubagne (FR); UNIVERSITE CLAUDE BERNARD - LYON 1, 69100 Villeurbanne (FR); Institut National des Sciences Appliquees de Lyon (Insa Lyon), 69100 Villeurbanne (FR); Ecole Superieure de Chimie, Physique, Electronique de Lyon, 69616 Villeurbanne Cedex (FR); Centre National de la Recherche Scientifique (CNRS), 75016 Paris (FR)
(72) Inventor: Gay, Isabelle, 13124 Peypin (FR); Dufour, Alexandre, 69006 Lyon (FR); Marquette, Christophe, 69100 Villeurbanne (FR); Petiot, Emma, 69100 Villeurbanne (FR); Barbaroux, Magali, 13112 La Destrousse (FR)
(74) Representative: Derambure Conseil

(57) **Abstract**

The invention concerns a sterile enclosure (91) comprising a container (92) and a printing needle holder device (58) fixed to the container. The container comprising a base wall (92), a side wall (94), the side wall and the base wall delimiting an interior chamber, one inlet port and outlet port and an aperture (24). The printing needle holder device (58) comprising a needle adapter, a printing needle and a flexible sheath having a first end edge fixed to the needle adapter and a second end edge fixed to the aperture of the container, a three dimensional biological structure being intended to be printed on the base wall (92), the interior chamber being intended to receive a cell culture media.

## Description

### Field of the invention

The present invention relates to a printing needle holder device and a sterile enclosure for printing and accommodating a biological three-dimensional structure. The invention also concerns a method of printing and culturing biological cells

### Technical background

It is known to print out biological three-dimensional structures onto a printing platform of a three-dimensional printers. After the structure is formed, the printed components are immersed in a media for a cell/culture process. So once printed, the biological three- dimensional structure must be transferred to a separate bioreactor for the cell/culture process. However, it is difficult to maintain the sterile conditions during the transfer of the biological three-dimensional structure to the bioreactor.

To avoid this difficulty, it is also known to print out the biological three-dimensional structure in a container adapted to contain and control a culture media and which also comprises a printer or a movable robotic arm equipped with a printing head. After printing, it is no more necessary to transfer the biological three- dimensional structure in a separate bioreactor for the cell/culture process.

A drawback of this operating mode is that the container is cumbersome and heavy since it must contain the printer or the movable robotic arm. As a consequence, when the biological three-dimensional structure has been formed, the printer or the movable robotic arm equipped with the printing head cannot be used to print out another structure because the container must be kept hermetically closed during the cell/culture process. Due to its size and its mechanical part which are greased, it can be problematic to sterilise the printer or the movable robotic arm and to keep it sterilized. As the container is large and cumbersome, it can be hard to move it to place it in another room during the cell/culture process.

### Object and summary of the invention

A first purpose of the present invention is to propose a printing needle holder device that can be fixed to different containers which can have different size or different properties which are specific to the biological structure to be printed.

A second purpose of the present invention is to propose a printing needle holder device that can be fixed both to a single use sterile enclosure for example for an industrial use or to an enclosure that can sterilized and used several times for example for a laboratory use for experimental purposes.

A third purpose of the present invention is to propose a sterile enclosure that is less bulky, cumbersome and heavy. Since the printer or a part of it is not comprised in the sterile enclosure, its size and its weight are significantly reduced.

A fourth purpose of the present invention is to propose a sterile enclosure that can be easily moved and stored after the printing process.

A fifth purpose of the present invention is to propose a sterile enclosure which allows immediate reuse of the printer after a biological three-dimensional structure has been formed.

A sixth purpose of the present invention is to propose a sterile enclosure which allows an easy displacement of the printing head in at least two perpendicular directions, and in particular in three perpendicular directions without generating stress on the needle and without risk of bending or breaking of the needle. As a consequence, the shape of the biological three-dimensional structure can be better controlled. Another consequence is that the printing platform does not need to be movable along a vertical direction because the printing needle holder device allows an easy move of the printing head along this direction. Accordingly, a less complex printer can be used. In the same way, the printer can use a smaller motor because there less resistance is applied to the robotic arm.

A seventh purpose of the present invention is to propose a sterile enclosure which is modular. Since different type of printing needle holder can be fixed to different type of container, the sterile enclosure can be adapted to different uses and needs.

A eighth purpose of the present invention is to propose a sterile enclosure which simplifies both the printing of a biological three- dimensional structure and its culture in a single container.

A ninth purpose of the present invention is to propose a method of printing and culturing a three-dimensional biological structure that ensures that there will be no contamination of the printed biological tree-dimensional structure with the bioink remaining in the needle after printing.

These purposes have been reached by a printing needle holder device, a sterile enclosure and a method as defined in the independent claims. Secondary features of the printing needle holder device and of the sterile enclosure are set out in the dependent claims.

### Brief description of figures

Fig. 1 is a perspective and schematic view of a printing needle holder device according to a first embodiment of the present invention;
Fig. 2 is a perspective and schematic view of a printing needle holder device according to a second embodiment of the present invention;
Fig. 3 is a perspective and schematic view of a printing needle holder device according to a third embodiment of the present invention;
Fig. 4 is a perspective and schematic view of a printing syringe in a holder and of a printing needle holder device according to a fourth embodiment of the present invention;
Fig. 5 is a perspective and schematic view of a printing needle holder device according to a fifth embodiment of the present invention;
Fig. 6 is a perspective and schematic view of a printing needle holder device according to a seventh embodiment of the present invention;
Fig. 7 is a perspective and schematic view of a container and a schematic view of a printing needle holder of a sterile enclosure according to a first embodiment of the invention;
Fig. 8 is a perspective and schematic view of a container and a schematic view of a printing needle holder of a sterile enclosure according to a second embodiment of the invention;
Fig. 9 is a perspective and schematic view of a container and a schematic view of a printing needle holder of a sterile enclosure according to a third embodiment of the invention;
Fig. 10 is a perspective and schematic view of a sterile enclosure according to a fourth embodiment of the invention;
Fig. 11 is a perspective and schematic view of a sterile enclosure according to a fifth embodiment of the invention;
Fig. 12 is a is a flowchart representing the steps of the method according to the present invention.

### Detailed description of the invention

In the below description, the term "printing" means making a manual deposit or injection of bioink composition in a sterile enclosure and making a 3D-printing with a displaceable robotic arm.

The printing needle holder device 2 according to the invention is intended to be fixed to a container 4 to form a sterile enclosure 6. Different examples of container will be described in the below description.

The printing needle holder device 2 according to the invention is also intended to be fixed to a printing cartridge or a barrel of a printing syringe 8 for the printing operation.

The printing syringe or the printing cartridge may comprise a bioink composition. A bioink composition is a biomaterial capable of forming a hydrogel and a cell population in a controlled three-dimensional shape. An example of bioink is described in WO 2017115056.

For the printing operation, the printing syringe or cartridge can be mounted on a displaceable printer for example on a displaceable robotic arm.

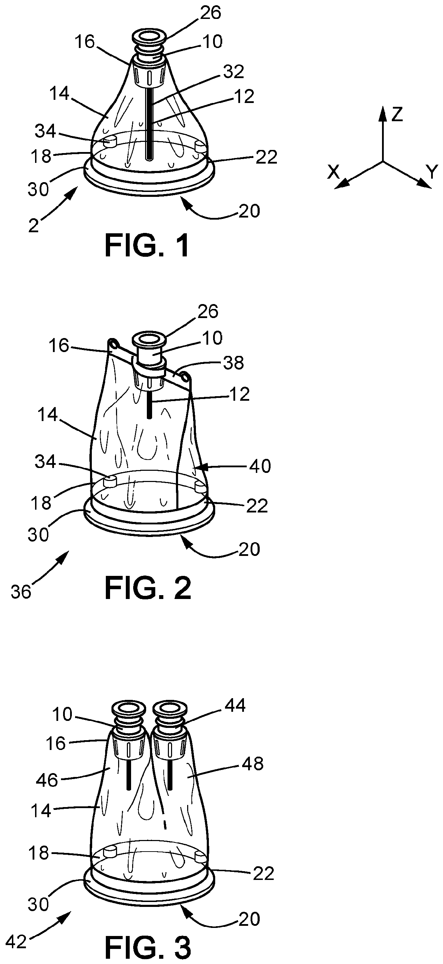

Referring to FIG. 1, the printing needle holder device 2 according to a first embodiment of the present invention comprises a needle adapter 10, a printing needle 12 hold by the needle adapter, and a flexible sheath 14 having a first end edge 16 tightly fixed to the needle adapter.

The needle adapter 10 here used is the tip that is usually connected to the open end of a barrel of a medical syringe. The needle adapter 10 comprises a needle hub holding the needle and a connector part configured to be connected to a corresponding connector part of the barrel of the syringe. The connector part and the corresponding connector part are for example a Luer lock connector.

Alternatively, the needle adapter 10 is a needle adapter which is integral with the printing cartridge.

Preferably, the printing needle 12 is a biological printing needle.

The flexible sheath delimits an internal cavity open to the outside. The flexible sheath comprises a first end edge 16 that is tightly fixed to the needle adapter and a second end edge 18 which delimits an opening open to the outside. The second end edge 18 is intended to be fixed to a container 4. Preferably, the second end edge is opposite to the first end edge as illustrated on the figures.

The flexible sheath 14 can be made in one material among a thermoplastic elastomer, a thermoset and a silicone.

In particular, the silicone can be one material among an unsaturated rubbers that can be cured by sulfur vulcanization, a Natural polyisoprene: cis-1,4-polyisoprene natural rubber (NR) and trans-1,4-polyisoprene gutta-percha, a Synthetic polyisoprene (IR for isoprene rubber), a Polybutadiene (BR for butadiene rubber), a Chloroprene rubber (CR), a polychloroprene, a Neoprene, a Baypren etc., a Butyl rubber (copolymer of isobutene and isoprene, IIR) Halogenated butyl rubbers (chloro butyl rubber: CIIR; bromo butyl rubber: BIIR), a Styrene-butadiene rubber (copolymer of styrene and butadiene, SBR) a Nitrile rubber (copolymer of butadiene and acrylonitrile, NBR) also called Buna N rubbers, a Hydrogenated nitrile rubbers (HNBR) Therban and Zetpol, (Unsaturated rubbers can also be cured by non-sulfur vulcanization if desired.), a Saturated rubbers that cannot be cured by sulfur vulcanization, EPM (ethylene propylene rubber, a copolymer of ethene and propene) and an EPDM rubber (ethylene propylene diene rubber, a terpolymer of ethylene, propylene and a diene-component), an Epichlorohydrin rubber (ECO), a Polyacrylic rubber (ACM, ABR), a Silicone rubber (SI, Q, VMQ), a Fluorosilicone rubber (FVMQ), a Fluoroelastomers (FKM, and FEPM) Viton, a Tecnoflon, Fluorel, an Aflas and Dai-El, a Perfluoroelastomers (FFKM) Tecnoflon PFR, a Kalrez, a Chemraz, a Perlast, Polyether block amides (PEBA), a Chlorosulfonated polyethylene (CSM), (Hypalon) an Ethylene-vinyl acetate (EVA) and a Thermoplastic elastomers (TPE).

In the first embodiment illustrated on Figure 1 which is a non-limiting embodiment, the flexible sheath 14 is made with a thermoplastic elastomer that is welded to the needle adapter.

The flexible sheath forms 14 a flexible sleeve.

In the illustrated embodiment, the flexible sheath forms 14 a skirt that extends around the needle adapter. The flexible sheath has here a general flare shape. Alternatively, the flexible sheath 14 may have a cup shape, a belt shape, a tube shape or a folding tube shape.

Advantageously, the sheath authorizes the shift of the needle along a vertical direction. As consequence, it no more necessary to use a printer with a vertically movable printing plate-form.

Advantageously, the sheath authorizes the shift of the needle in two horizontal and perpendicular directions with respect to the container which is in a stationary position on the printing platform during printing process of a three-dimensional biological structure in the sterile enclosure.

When the needle holder device is fixed to a rigid container, the dimension of the opening is at least as large as the size of the biological structure to be printed. When the needle holder device is fixed to a flexible container, the opening can have a smaller size of the biological structure to be printed since the flexibility of the container will be used to authorize the movement of the needle.

Preferably, the second end edge 18 delimits an opening 20 which is at least twice larger than the opening delimited by the first end edge which is fixed to the needle adapter.

Preferably, the printing needle 12 is a biological printing needle. The needle adapter 10 and the printing needle 12 form a printing head.

The printing needle holder device 2 according to the first embodiment further comprises a rigid flange 22 fixed to the second end edge 18. In the illustrated embodiment, the rigid flange 22 presents an annular shape. The rigid flange can be circular, rectangular or quadrat. The rigid flange can, for example, be made of plastic or stainless steel.

The rigid flange 22 is intended to be fixed to an aperture 24 of the container 4. To this end, the rigid flange 22 can advantageously be provided with a first connector part 26 conform to be fixed to a complementary connector part of a rigid flange 28 of the container, as described below. The first connector part 26 could be an aseptic connector or a non-aseptic connector. It may involve a clamp system in particular a tri-clamp system.

Alternatively, the rigid flange 22 can be fixed to the aperture 24 of the container by any fixation means such as for example an attachment link, a rubber etc. The rigid flange 22 can also be welded to a flexible film of the container 4 as described below. In the embodiment illustrated on figure 1, the needle adapter 10 comprises a second connector part 30 adapted to be fixed to a complementary connector part of a syringe or of a printing cartridge. The second connector part 30 could be an aseptic connector or a non-aseptic connector. It may involve a clamp system in particular a tri-clamp system.

Advantageously, the printing needle holder device 2 illustrated on figure 1 comprises a needle protection cover 32 adapted to be snap on an abutment ring of the needle adapter 10. The needle protection cover avoids that the drilling of the flexible sheath by the needle.

Advantageously, the printing needle holder device 2 can comprise a receiving sleeve 34 adapted to receive and hold the printing needle 12 before or after printing. In the embodiment illustrated on figure 1, the receiving sleeve 34 is integral with the rigid flange 22.

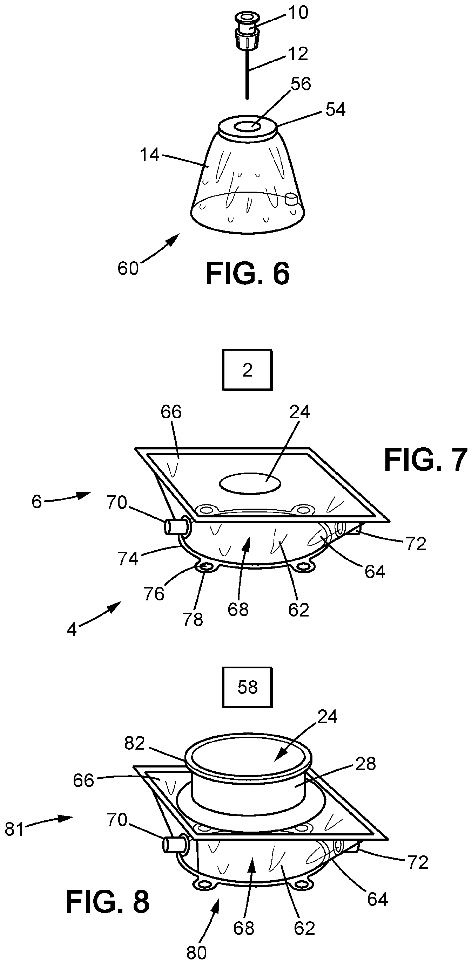

Alternatively, the receiving sleeve 34 is integral with the flexible sheath as illustrated on Figure 6. In this later embodiment, the receiving sleeve is preferably made with the same material as the flexible sheath.

Referring to FIG. 2, the printing needle holder device 36 according to a second embodiment of the present invention is similar to the printing needle holder device according to the first embodiment at the exception of the fact that the flexible sheath presents a different shape. The technical elements of the second embodiment identical to the technical elements of the first embodiment have been referenced by the same references and will not be described again. In this second embodiment, the needle adapter 10 is fixed between two junctions 38 of the first end edge 16 of the flexible sheath. At each junction, a part of the first edge 16 of the flexible sheath is fixed to another part of the first end edge to make a gusset 40.

Referring to FIG. 3, the printing needle holder device 42 according to a third embodiment of the present invention is similar to the printing needle holder device according to the first embodiment at the exception of the fact that the flexible sheath 14 presents two sets of needle adapters 10, and needles. The technical elements of the second embodiment identical to the technical elements of the first embodiment have been referenced by the same references and will not be described again.

In this embodiment, the printing needle holder device 42 comprises two needle adapters 10, 44 holding two printing needles12. To this end, the flexible sheath 10 presents two peaks 46, 48 or fingers at its upper side. One portion of the first end edge 16 is assembled or moulded to form the first peak 46 which is tightly fixed to the first needle adapter 10. In the same way, another portion of the first end edge 16 is assembled or moulded to form the second peak 48 and is tightly fixed to a second needle adapter 44.

Preferably, this embodiment is made by moulding a silicone.

Alternatively, the printing needle holder device 42 can comprise three, four or five sets of needle adapter and needle.

This embodiment can for example be used when performing several printing steps of the same bioink separated by break times. For example, the cells can be cultured during these break times. Advantageously, a different needle and needle adapter is used for each printing step to avoid a contamination of the already printed three dimensional- structure by the residual material which remains in the needle.

This embodiment can also be used when performing several printing steps with different bioinks for printing different types of cells or tissues for instance muscle and skin cells. In this case, different bioink are introduced in each printing needle 12 and needle adapter 10, 44.

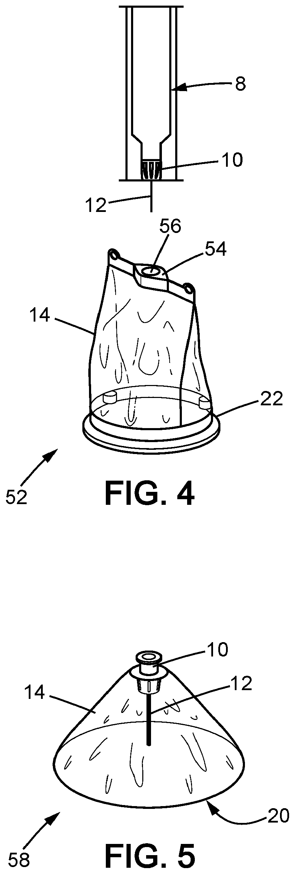

Referring to FIG. 4, the printing needle holder device 52 according to a fourth embodiment of the present invention comprises a rigid frame 54, a membrane 56 supported by the rigid frame, and a flexible sheath 14 tightly fixed to the rigid frame. The membrane 56 constitutes a septum 56 that is intended to be punctured by a printing needle of a syringe as illustrated on figure 4. For example, the syringe is disposed in a casing of a movable robotic arm.

In the non-limiting embodiment illustrated on figure 1, the rigid frame 54 has as a general oblong shape. The membrane 56 is tightly sealed to the interior face of the rigid frame. The membrane is configured so that it can be punctured. To this end, the membrane is made in a material usually used for making septum.

In the illustrated embodiment, the flexible sheath 14 presents the same shape as the printing needle holder device of the second embodiment. The flexible sheath can also have the same shape as the printing needle holder device of the first embodiment or the shape of the third embodiment with several rigid frames and septum.

As concern the other properties, the flexible sheath 14 of the device according to the fourth embodiment is similar to the flexible sheath 14 of the device according to first embodiment. It will not be described again.

Advantageously, the printing needle holder according to this embodiment also authorizes the displacement of the rigid frame and of the printing needle in two perpendicular horizontal directions as well as in a vertical direction during a printing of a three-dimensional biological structure in the container.

Referring to FIG. 5, the printing needle holder device 58 according to a fifth embodiment of the present invention is similar to the printing needle holder device 2 according to the first embodiment at the exception of the fact that it does not comprise a rigid flange.

The other technical elements of the fifth embodiment identical to the technical elements of the first embodiment have been referenced by the same references and will not be described again. The printing needle holder device 58 according to this embodiment is particularly adapted to be fixed to a container comprising a rigid flange, as described below.

Referring to FIG. 6, the printing needle holder device 60 according to a sixth embodiment of the present invention is similar to the printing needle holder device according to the first embodiment at the exception that :
- the needle adapter 10 has been replaced by a rigid frame 54 supporting a septum 56 and,
- the printing needle holder device 60 does not comprise a rigid flange fixed to the second end edge of the flexible sheath.

The other technical elements of the fifth embodiment identical to the technical elements of the first embodiment have been referenced by the same references and will not be described again.

The printing needle holder device according to the invention can be fixed to any existing container to form a sterile and hermetically closed enclosure. The invention also concerns a sterile enclosure. This sterile enclosure comprises different container that are particularly suitable to be fixed to the printing needle holder device according to the invention.

The sterile enclosure 6 according to a first embodiment of the present invention comprises a container 4 illustrated on figure 7 and a printing needle holder device 2 represented schematically.

The container 4 can be fixed to any printing needle holder device as illustrated on figures 1 to 4 to form a sterile and hermetical enclosure. The sterilisation process is performed after the fixation for example by gamma irradiation.

The container 4 comprises a base wall 62, a side wall 64, a top wall 66 and an aperture 24 adapted to be fixed to the rigid frame 22 of any printing needle holder device as illustrated on figures 1 to 4.

The side wall of the container can be a unique side wall for instance when the container has a cylindrical shape or several side walls for instance when the container has a parallelepiped shape. In this patent application, the term "a side wall" will be used to designate these two possibilities in order to simplify the comprehension of the description. The base wall and the side wall can be made from one or several plastic sheet(s).

The side wall 64, the base wall 62, the top wall 66 and the printing needle holder device delimit an interior chamber 68. The fixation between the container 4 and the rigid frame 22 can be done by any fixation means for example by welding or by an attachment link. The fixation must close hermetically the interior chamber.

The three-dimensional biological structure is intended to be printed on the base wall. The sterile enclosure obtained by the fixation of the container 4 to a needle holding device is intended to receive a cell culture media in the interior chamber.

In the illustrated embodiments, the top wall 66 comprises the aperture 24. The printing needle holder device close this aperture 24.

Alternatively, a side wall 64 can comprise an aperture instead of the top wall.

In this illustrated embodiment, the base wall 62, the side wall 64 and the top wall 66 are made with a flexible film. The flexible film can be made of a thermoplastic elastomer or of a thermoset.

The flexible film is generally composed of a multilayer film comprising a contact layer which in contact with the medium that fills the container, a barrier layer and an outer layer which is in contact with the external environment. The three layers are connected one to each other with a tie layer. If the container is to be filled with a biopharmaceutical product, the contact layer should be made from a material that can be in contact with this biopharmaceutical product without causing degradation of the film and of the biopharmaceutical product. Furthermore, it must be sealable on itself. For that purpose, the material is generally selected from polyolefins, such as polyethylene.

The barrier layer provides a barrier to the passage of gases such as oxygen, carbon dioxide and is typically made from ethylene vinyl alcohol (EVOH).

The outer layer contributes to the mechanical strength of the wall. For that purpose, it must be sufficiently flexible to withstand high mechanical stress but not be too much stretchable in order to prevent deformation of the enclosure when it is filled with a product.

The outer layer could comprise a polyolefin alone or in mixture with a copolymer of ethylene and a-olefin.

The side wall 64 and the base wall 62 are realised with the same sheet. Alternatively, the side wall 64 and the base wall 62 are realised with two different sheets welded one to the other.

Advantageously, the container 4 is flexible so that if necessary, the side wall 64 can move to follow the movement of the syringe during the printing process.

The container 4 comprises an inlet port 70 and an outlet port 72 for circulating a culture media after the printing process to culture the biological three-dimensional structure.

Preferably, the container 4 comprises supplementary ports not illustrated. The supplementary ports are used for fixing sensors for controlling different parameters of the culture media like for example its temperature.

Preferably, the container 4 comprises an extension 74 protruding from the container and extending in a horizontal plane. The extension comprises reliefs 76 to reference the position of the container with respect to a support and in particular with respect to a printing platform.

In the illustrated and non-limiting embodiment, an internal face of an edge of the flexible side wall is fixed to the internal face of a peripheral edge of the base wall 10 to form the extension 74.

The extension 74 protrudes from the base wall 10 in a horizontal plane.

In the illustrated embodiment, the extension 74 is provided with four perforated tabs 78 regularly spaced around the base wall. The holes of these tabs are adapted to be placed in four reliefs made on the printing platform to reference the position of the sterile enclosure 6.

Advantageously, the sterile enclosure 6 can be removed from the printing platform after a first cycle of printing to be place in another place for the cell/culture process. Afterwards, the sterile enclosure can be place again on the printing platform for a second printing cycle at the same position than for the first cycle of printing. A correct positioning is important for printing a biological three-dimensional structure because this one must be precisely shaped.

Alternatively, the extension 74 comprises only two reliefs 76 which have a circular arc shape. The reliefs 76 are through holes.

Advantageously, the extension 74 also allows to stabilize the container on a support because they extend in a horizontal plane.

Advantageously, the sterile enclosure 6 according to this first embodiment, can be placed in a rigid receptacle. The latter one does not need to be sterilized.

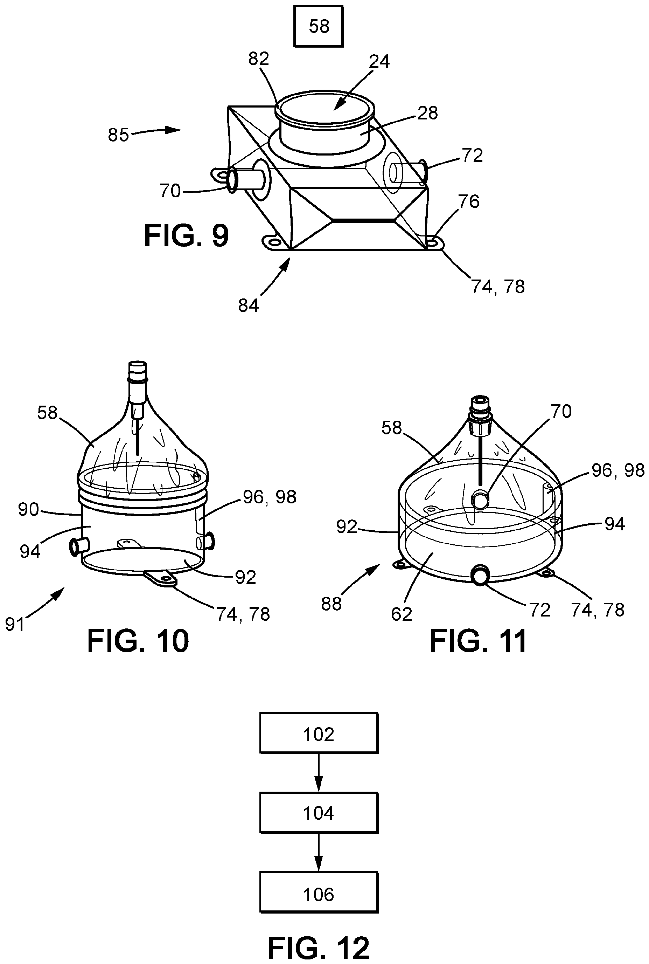

On the illustrated embodiment, the container 4 has a general shape of an inverted truncated pyramid. It can have any other shape, like for example a parallelepiped shape as illustrated on figure 9 or an inverted truncated cone shape.

In a similar manner, the side wall 64 can have any section among a circular section, a parallelepiped section, a square section and a rectangular section.

The side wall 64 can be made with a single sheet or with several sheets welded one to another.

The container 4 can also comprise a base stiff framework which is fixed to the base wall made of flexible film to form a flat surface. The base stiff framework has not be represented on the illustrated embodiment.

Referring to figure 8, the sterile enclosure 81 according to a second embodiment of the present invention comprises a container 80 and a printing needle holder device 58 represented schematically.

Alternatively, the sterile enclosure 81 can be made by the fixation of the printing needle holder device 60 illustrated on the figure 6 to the container 80 illustrated on figure 8 by any fixation element.

The container 80 is similar to the container illustrated on figure 7 at the exception that it further comprises an upper rigid flange 28 tightly fixed to the top wall 66 of the container around the aperture 24.

The technical elements of the sterile enclosure of the second embodiment identical to the technical elements of the first embodiment have been referenced by the same references and will not be described again.

Alternatively, the printing needle holder device illustrated on the figures 1 to 4 can also be fixed to the container 80 illustrated on figure 8. In this case, the rigid flange 28 comprises preferably a connector part 82 adapted to be fixed to the first connector part 26 of the printing needle holder device.

Advantageously, the first connector part 26 of the rigid flange 22 of the printing holder device and the connector part 82 of the container comprise both a clamp system in particular a tri-clamp system. Thus, the printing holder device and the container can be separately sterilized and whenever necessary fixed together.

The connector part 82 could also be a non-aseptic connector.

The rigid flange 28 is for example made in plastic.

The rigid flange 28 can be provided with a septum which close hermetically the aperture 24 of the container before its fixation to the needle holder device.

Referring to figure 9, the sterile enclosure 85 according to a third embodiment of the present invention comprises a container 84 and a printing needle holder device 58 represented schematically.

The container 84 is similar to the container illustrated on figure 8 at the exception that it presents a parralepipedal shape.

The technical elements of the sterile enclosure of the third embodiment identical to the technical elements of the first embodiment have been referenced by the same references and will not be described again.

The printing needle holder device 60 illustrated on figure 6 can also be fixed to the container 84 illustrated on figure 9.

Referring to figure 10, the sterile enclosure 91 according to a fourth embodiment of the present invention comprises a container 90 having a rigid base wall 92, a rigid side wall 94 and a printing needle holder device 58 conform to the fifth embodiment. In this embodiment, the container 90 does not comprise a top wall. The second end edge 18 of the needle holder device is tighly fasten to the top part of the side wall. The printing needle device 58 forms the whole top wall of the sterile enclosure. In the illustrated embodiment, the printing needle holder device 58 is tightly fixed to the container 90 with an attachment link.

In the illustrated embodiment, the side wall 94 has a cylinder shape. The extension 74 comprises four opposite perforated tabs 78 which project horizontally from the base wall to reference the position of the sterile enclosure.

Advantageously, the interior face of the side wall is provided with a receiving sleeve 96 conform to receive the printing needle 12 before or after printing operation. The receiving sleeve 96 is preferably arranged against the side wall.

Preferably, the interior face of the side wall comprises a vessel 98. The vessel is used to collect residual bioink during a priming phase for regulating the rate of flow of the printing needle. The vessel is also used for vessel purging the residual bioink in the needle. After printing, the residual bioink which remains in the needle can advantageously be purged in the vessel. To simplify the figures 10 and 11, the vessel has been represented by the same element as the receiving sleeve 96. Advantageously, the side wall 94 are rigid so that they can't fall in the printed three-dimensional structure and damage it.

When using a rigid container, the opening 20 delimited by the second end edge 18 of the printing needle holder has at least the dimension of the opening of the container, In a similar way, the opening 20 delimited by the second end edge 18 of the printing needle holder has at least the dimension of the biological structure to be print. Referring to figure 11, the sterile enclosure 88 according to a fifth embodiment of the present invention is similar to the sterile enclosure 91 according to the fourth embodiment of the present invention at the exception that the container 92 has a flexible base wall 62 and a rigid side wall 94. In this embodiment, the flexible film of the base wall is fixed to a cylindrical side wall to form the container. Advantageously, the flexible film of the base wall is attached to the lower edge of the rigid side walls. Thus, advantageously, the flexible film stays flat during the printing process.

The other technical elements of the fifth embodiment identical to the technical elements of the fourth embodiment have been referenced by the same references and will not be described again.

According to another non-illustrated embodiment, the base wall is rigid, and the side wall is fixed to the base wall to form the interior chamber.

The invention also concerns a method of printing and culturing a three-dimensional biological structure in a sterile enclosure conform to any of the above-described embodiments. The method comprises begins with an initial step during which
- the needle adapter 10 of a printing needle holder device according to the embodiments illustrated on figures 1 to 3 and 5 is fixed to a printing cartridge or a syringe, or
- the septum 56 of a printing needle holder device according to the embodiments illustrated on figures 5 and 6 is punctured by a printing syringe 8.

At a step 102, the three-dimensional biological structure is printed, the flexible sheath authorizes the move of the needle adapter 10 and of the needle in three perpendicular directions while keeping the sterility of the interior chamber,

At a step 104, a cell culture media is supplied to the interior chamber,

At a step 106, the flexible sheath is assembled and fixed together to separate the biological printing needle from the interior chamber of the bioreactor to avoid a contamination of the printed three- dimensional structure by the residual bioink remaining in the needle.

Preferably, the fixation is performed by welding two opposite part of the flexible enclosure. A clamp can also be used to separate two parts of the flexible enclosure.

## Claims

1. Printing needle holder device (2,36,42,58) adapted to be fixed to a container (4) having an aperture (24), said printing needle holder device comprising :
- at least one needle adapter (10),
- at least a printing needle (12) extending in a defined direction (Z), the at least one needle adapter holding the printing needle (12),
- a flexible sheath (14) having a first end edge (16) tightly fixed to the at least one needle adapter and a second end edge (18) delimiting an opening (20) intended to be fixed to the aperture (24) of the container,
the printing needle holder (2) authorizing the displacement of the printing needle (12) in at least two perpendicular directions (X Y) when the second end edge (18) is fixed to the container (4) for printing a three-dimensional biological structure in the container, the two perpendicular directions (X, Y) being perpendicular to the defined direction (Z).

2. Printing needle holder device (52,60) adapted to be fixed to a container (4) having an aperture (24), said printing needle holder device comprising :
- at least one rigid frame (54) and a septum (56) supported by the rigid frame, the septum being designed to be punctured by a printing needle (12),
- a flexible sheath (14) having a first end edge (16) tightly fixed to the at least one rigid frame (54) and a second end edge (18) delimiting an opening (20) intended to be fixed to the aperture (24) of the container,
the printing needle holder (52, 60) authorizing the displacement of the printing needle (12) in at least two directions (X, Y) when the second end edge (18) is fixed to the container for printing a three-dimensional biological structure in the container, the two perpendicular directions (X, Y) being in a plane parallel to the septum.

3. Printing needle holder device (2,36,42,52,58, 60) according to claims 1 and 2, which comprises a rigid flange (22) fixed to the second edge (18), the rigid flange being intended to be fixed to the aperture (24) of the container.

4. Printing needle holder device (2,36,42,52,58, 60) according to claim 3, wherein the rigid frame (22) is provided with a first connector part (26) intended to be fixed to the container.

5. Printing needle holder device (2,36,42,52,58,60) according to any of claims 1 to 4, wherein the at least one flexible sheath (14) is made of one material among a thermoplastic elastomer, a thermoset and a silicone.

6. Printing needle holder device (2,36,42,52,58,60) according to claims 1 to 5, which comprises a receiving sleeve (34) adapted to receive the needle.

7. Printing needle holder device (52,60) according to any of claims 1, 3 to 6, which comprises at least one needle protection cover (32) adapted to be snap on the needle adapter (10).

8. Printing needle holder device (52,60) according to any of claims 1, 3 to 7 wherein the at least one needle adapter (10) comprises at least one second connector part (30) conform to be connected to a complementary connector part of a printing cartridge.

9. Printing needle holder device (52,60) according to any of claims 1, 3 to 7, which comprises a printing cartridge integral with the at least one needle adapter (10).

10. Sterile enclosure (6,91,88) comprising a container (4,90, 92) said container comprising:
- a base wall (62,92),
- at least one side wall (64,94), the at least one side wall and the base wall delimiting an interior chamber (68),
- at least one inlet port (70) and at least one outlet port (72),
- an aperture (24),
**characterized in that** it comprises a printing needle holder device (2,36,42,52,58,60) conform to any claims 3 to 9, a rigid flange (22) of the printing needle holder device being fixed to the container to close the aperture; the three dimensional biological structure being intended to be printed on the base wall (62), the interior chamber (68) being intended to receive a cell culture media.

11. Sterile enclosure (81, 85) comprising a container (80, 84), said container comprising :
- a base wall (62,92),
- at least one side wall (64,94), the at least one side wall and the base wall delimiting an interior chamber (68),
- at least one inlet port (70) and at least one outlet port (72),
- an upper rigid flange (28) delimiting an aperture (24),
**characterized in that** it comprises a printing needle holder device (2,36,42,58) conform to any claims 1, 4 to 9, the upper rigid flange (28) being fixed to an opening (20) of the printing needle holder device, the three dimensional biological structure being intended to be printed on the base wall (62,92), the interior chamber (68) being intended to receive a cell culture media.

12. Sterile enclosure (6, 81, 85,91,88) according to any of claims 10 and 11, wherein the base wall (62) and the at least one side wall (64) are made with a flexible film.

13. Sterile enclosure according to any of claims 10 and 11, wherein the base wall (92) is rigid and the at least one side wall (94) is made with a flexible film.

14. Sterile enclosure (91) according to any of claims 10 and 11, wherein the base wall (62) is made with a flexible film and the at least one side wall (64) is rigid.

15. Sterile enclosure (88) according to any of claims 10 and 11, wherein the base wall (92) and the at least one side wall (94) are rigid.

16. Sterile enclosure (6, 81, 85,91,88) according to any of claims 10 to 15, which comprises a receiving sleeve (96) for receiving the printing needle.

17. Sterile enclosure (6, 81, 85,91,88) according to any of claims 10 to 15, which comprises a vessel (98) for priming the flow of bioink in the printing needle and/or for purging the printing needle.

18. Sterile enclosure (6, 81, 85,91,88) according to any of claims 10 to 16, wherein the base wall (62) comprises at least one extension (74) protruding from the base wall in a horizontal plane.

19. Sterile enclosure (6, 81, 85,91,88) according to claim 18, wherein the extension (74) comprises reliefs to reference the location of the enclosure with respect to a support, for example a printing platform.

20. Sterile enclosure (6, 81, 85,91,88) according to the combination of claims 1, 3 and 10 wherein the rigid flange (22) of the printing needle holder device is fixed to the upper rigid flange (28) of the container.

21. Sterile enclosure (6, 81, 85,91,88) according to claim 19, which comprises a septum fixed to the upper rigid flange.

22. Method of printing and culturing a three dimensional biological structure in a sterile enclosure (6, 81, 85,91,88) conform to any of claims 9 to 21, the method comprising the successive following steps:
- printing (102) of the three dimensional biological structure, the flexible sheath authorizing the move of the needle adapter and the printing needle in at least two perpendicular directions during the biological printing step while keeping the sterility of the interior chamber,
- supplying (104) a cell culture media to the interior chamber (68).

23. Method of printing according to claim 22 which further comprises a fixation (106) of the flexible sheath, for example by welding, to separate the printing needle (12) from the interior chamber.
